# EUROPEAN PATENT APPLICATION

(11) **EP 4 393 474 A1**
(43) Date of publication of application: **03.07.2024**
(21) Application number: 23220229.1
(22) Date of filing: 26.12.2023
(51) Int. Cl.: A61K 9/00, A61K 9/20, A61K 9/28, A61K 31/4965, A61P 9/12

(54) **A FORMULATION COMPRISING SELEXIPAG**

(30) Priority: 28.12.2022 TR 202220983
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); PEHLIVAN AKALIN, Nur, Istanbul (TR); TUNC, Guldeniz, stanbul (TR)
(74) Representative: Sevinç, Erkan

(57) **Abstract**

The present invention relates to a tablet formulation comprising selexipag form III, wherein selexipag form III has a d (0.9) particle size between 1 µm to 150 µm. Furthermore, the tablet is obtained using an effective process.

## Description

### Field of the Invention

The present invention relates to a tablet formulation comprising selexipag form III, wherein selexipag form III has a d (0.9) particle size between 1 µm to 150 µm. Furthermore, the tablet is obtained using an effective process.

### Background of the Invention

Selexipag is prostacyclin receptor agonist that causes vasodilation in pulmonary vasculature and is used in the therapy of pulmonary arterial hypertension (PAH). Selexipag also known as 2-(4-((5,6-diphenylpyrazin-2-yl)(isopropyl)amino)butoxy)-N-(methylsulfonyl)acetamide can be represented by the following chemical structure according to Formula I:

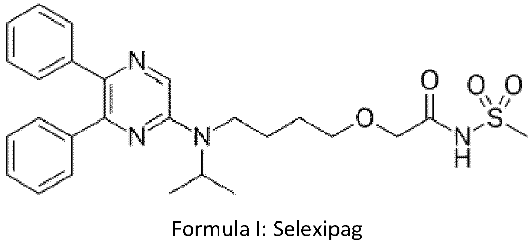

Depending on the dose strength, each round film-coated tablet for oral administration contains 200, 400, 600, 800, 1000, 1200, 1400, or 1600 mcg of Selexipag. The Uptravi^{®} film-coated tablet is a standard immediate-release tablet that contains D-mannitol, corn starch, low substituted hydroxypropylcellulose, hydroxypropylcellulose, and magnesium stearate. The tablets are film coated with a coating material containing hypromellose, propylene glycol, titanium dioxide, carnauba wax along with mixtures of iron oxide red, iron oxide yellow or iron oxide black.

Selexipag is considered to be a BCS (Biopharmaceutics Classification System) Class II drug, i.e. having high permeability and low solubility.

Different salts and solid state forms of an active pharmaceutical ingredient may possess different properties. Such variations in the properties of different salts and solid state forms and solvates may provide a basis for improving formulation, for example, by facilitating better processing or handling characteristics, improving the dissolution profile, or improving and shelf-life. These variations in the properties of different salts and solid state forms may also provide improvements to the final dosage form, for instance, if they serve to improve bioavailability.

WO 2010/150865 A1 discloses three crystalline forms of selexipag, designated form I, form II and form III. The crystalline forms of WO 2010/150865 are described as useful for the preparation of pharmaceutical formulations and oral dosage forms, such as tablets comprising crystalline selexipag, are described. It should be noted that amorphous selexipag was only described in one single experiment using 1,3,5-trimethylbenzene as solvent in WO 2010/150865.

EP3481807 (A1) discloses novel crystalline forms of selexipag and its process for the preparation thereof.

In prior art, there are also several patents which disclose selexipag in oral pharmaceutical dosage forms. However, despite the dissolution problem of selexipag, an effective formulation and method has not been disclosed.

There still remains a need in the art to provide an improved a tablet formulation comprising selexipag or crystalline polymorph thereof having high solubility, excellent physicochemical properties and accordingly a high bioavailability and a long-term stability.

### Detailed Description of the Invention

The main object of the present invention is to provide a tablet formulation comprising selexipag form III with having the desired level of dissolution rate and high stability and excellent physicochemical properties, such as flowability, compressibility, homogeneity, and content uniformity which overcomes the above-described problems in the prior art and have additive advantages over them.

Another object of the present invention is to provide a process for preparing a tablet formulation comprising selexipag form III. The process is a simple, rapid, cost effective, time-saving, and industrially convenient method.

As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

We found that when we used selexipag form III in our formulation, we created a stable formulation with good physicochemical properties. Selexipag form III has high melting point, high glass transition temperature. It is also nonhygroscopic. But, it has low solubilty. So, we found the importance of particle size of selexipag form III to overcome low solubility in the formulation.

According to one embodiment of this invention, a tablet formulation comprises selexipag form III, wherein selexipag form III has a d (0.9) particle size between 1 µm to 150 µm. The selexipag having above described particle size provides the dissolution profile, also provides the improved flowability.

According to one embodiment of the present invention, selexipag form III has a d (0.9) particle size between 1 µm to 100 µm. Preferably, it is between 1 µm to 60 µm, or between 1 µm to 35 µm, or between 1 µm to 10 µm.

According to one embodiment of this invention, the amount of selexipag form III is between 0.05% and 10.0%, preferably between 0.05% and 5.0% by weight of the total tablet.

According to one embodiment of the present invention, the tablet formulation further comprises at least one pharmaceutically acceptable excipient selected from the group comprising binders, fillers, disintegrants, lubricants or mixtures thereof.

Suitable binders are selected from the group comprising hydroxypropyl cellulose, polyvinylpyrrolidone, sodium carboxymethyl cellulose, sodium carboxymethyl cellulose, polyethylene glycol, pregelatinized starch, sodium alginate, hydroxypropyl methylcellulose, carboxy methyl cellulose, methyl cellulose, polymethacrylates, methacrylate polymers, polysaccharides, carbomer, poloxamer, polyacrylamide, polyoxyethylene-alkyl ether, polydextrose, polyethylene oxide or mixtures thereof.

Selexipag form III is used small proportion that can lead to considerable problems during the manufacture of the composition with regard to the uniformity of the content of active agent in the individual composition units. It reflects that content uniformity play important role in the dissolution of the drug. We found that the use of binders is important in formulation.

According to one embodiment of the present invention, the binder is hydroxypropyl cellulose.

According to one embodiment of the present invention, hydroxypropyl cellulose has a d (0.9) particle size between 15 µm to 250 µm.

According to one embodiment of the present invention, hydroxypropyl cellulose has a d (0.9) particle size between 25 µm to 230 µm, or between 40 µm to 210 µm, or between 50 µm to 190 µm, or between 60 µm to 170 µm, or between 70 µm to 150 µm.

According to one embodiment of the present invention, hydroxypropyl cellulose has a viscosity in a range of between 75 CPS and 150 CPS at room temperature (25°) in %5 water solution. The viscosity is measured by using RVDV-II or LVDV-II [GR1] Brookfield Digital Viscometers in the conditions of room temperature and 20 rpm. Time of stabilization is 30 seconds. Hydroxypropyl cellulose having above described viscosity provides the desired dissolution profile.

According to one embodiment of this invention, the amount of binder is between 1.0% and 8.0%, between 2.0% and 6.0% by weight of the total tablet.

Suitable fillers are selected from the group comprising corn starch, microcrystalline cellulose, lactose monohydrate, neutral pellets, calcium sulfate, cellulose, cellulose acetate, compressible sugar, ethylcellulose, fructose, glyceryl palmitostearate, lactitol, lactose, magnesium carbonate, magnesium oxide, maltodextrin, maltose, sodium alginate, sodium chloride, sorbitol, sucrose, sugar spheres, talc, tragacanth, trehalose, xylitol or mixtures thereof.

According to one embodiment of the present invention, fillers are D-mannitol and corn starch. Using mannitol (D-mannitol) and corn starch help to provide both the desired stability and excellent physicochemical properties.

According to one embodiment of this invention, D-mannitol having a specific surface area is 1.01 m²/g or more. Preferably, D-mannitol having specific surface area is between 1.01 m²/g and 1.19 m²/g, or between 1.22 m²/g and 2.25 m²/g, or between 2.26 m²/g and 4.20 m²/g. The use of d-mannitol with this specific surface area helped to provide the desired stability in the formulation.

According to one embodiment of this invention, the amount of filler is between 65.0% and 95.0%, between 78.0% and 90.0% by weight of the total tablet.

According to one embodiment of this invention, the amount of D-mannitol is between 40.0% and 65.0%, between 52.0% and 60.0% by weight of the total tablet.

According to one embodiment of this invention, the amount of corn starch is between 25.0% and 40.0% by weight of the total tablet.

Suitable disintegrants are selected from the group comprising sodium starch glycolate, low-substituted hydroxypropyl cellulose, croscarmellose sodium, crospovidone, carboxymethyl cellulose, sodium carboxymethyl cellulose, calcium carboxymethyl cellulose, sodium carboxymethyl starch, hydroxymethyl starch or mixtures thereof.

According to one embodiment of this invention, the disintegrant is sodium starch glycolate.

According to one embodiment of this invention, the amount of sodium starch glycolate is between 1.0% and 7.0% by weight of the total tablet.

Suitable lubricants are selected from the group comprising magnesium stearate, calcium stearate, sodium stearyl fumarate, potassium stearate, stearic acid, sodium chloride, sodium benzoate, sodium acetate, sodium oleate, polyethylene glycols or mixtures thereof.

According to one embodiment of the present invention, the lubricant is magnesium stearate.

According to one embodiment of this invention, the amount of lubricant is between 0.5% and 3.5% by weight of the total tablet.

According to one embodiment of the present invention, the tablet formulation is coated with film coating.

According to one embodiment of this invention, the tablet formulation comprises;
- Selexipag form III
- D-Mannitol
- Corn starch
- Hydroxypropyl cellulose
- Sodium starch glycolate
- Magnesium stearate.

According to one embodiment of this invention, the tablet formulation is obtained by wet granulation using solvent.

Suitable solvents are selected from the group comprising pure water, dichloromethane, 0.1N HCl, methanol, ethanol, isopropyl alcohol, propylene glycol, polyethylene glycol, glycerine, cyclomethicone, glycerine triacetate, diethylene glycol monoethyl ether or mixtures thereof. Preferably, the solvent is water.

Active substance used in low amounts cause some problems which are homogeneity and content uniformity. In this invention, to eliminate this problem, wet granulation is preferred in terms of physicochemical properties, such as flowability, compressibility and content uniformity of selexipag. Especially, using suitable solvent helps to provide the physicochemical properties properties.

According to one embodiment of this invention, a process for preparing a tablet formulation comprising selexipag form III comprises the following steps:
a) Mixing selexipag form III and hydroxypropyl cellulose,
b) Mixing corn starch and D-mannitol,
c) Then, mixing the mixture at step (a) and the mixture at step (b) with geometric blending,
d) Granulating the powder mixture at step (c) with pure water,
e) Drying wet granules until their moisture reaches 1-2%,
f) Adding sodium starch glycolate and then mixing,
g) Adding magnesium stearate,
h) Compressing the mixture into tablets,
i) Coating the tablets.

Prepared tablets are packed with the blister packaging material which comprises desiccant into the sealing layer of the blister. The selected packaging protectives against degradation of active substance. So, the packaging provides long term shelf life of the tablets.

### Example 1: A film coated tablet

| **Ingredients** | **Amount (% by weight of the total formulation)** |
|---|---|
| Selexipag form III | 0.05 - 10.0 |
| Mannitol | 40.0 - 65.0 |
| Corn starch | 25.0 - 40.0 |
| Hydroxypropyl Cellulose | 2.0 - 10.0 |
| Sodium starch glycolate | 1.0 - 7.0 |
| Magnesium stearate | 0.5 - 3.5 |
| **TOTAL** | **100** |

### Example 2: A film coated tablet

| **Ingredients** | **Amount (% by weight of the total formulation)** |
|---|---|
| Selexipag form III | 1.19 |
| Mannitol Parteck 100M | 52.74 |
| Corn starch | 35.56 |
| Hydroxypropyl Cellulose (Klucel LXF) | 4.0 |
| Sodium starch glycolate | 5.04 |
| Magnesium stearate | 1.5 |
| **Film coating** | **3.6** |
| **TOTAL** | **100** |

### Example 3: A film coated tablet

| **Ingredients** | **Amount (% by weight of the total formulation)** |
|---|---|
| Selexipag form III | 0.143 |
| Mannitol | 51.85 |
| Corn starch | 34.35 |
| Hydroxypropyl Cellulose | 3.88 |
| Sodium starch glycolate | 4.85 |
| Magnesium stearate | 1.4 |
| **Film coating** | **3.5** |
| **TOTAL** | **100** |

**A process for example 1 or 2 or 3;**
a) Mixing selexipag form III and hydroxypropyl cellulose,
b) Mixing corn starch and D-mannitol,
c) Then, mixing the mixture at step (a) and the mixture at step (b) with geometric blending,
d) Granulating the powder mixture at step (c) with pure water,
e) Drying wet granules until their moisture reaches 1-2%,
f) Adding sodium starch glycolate and then mixing,
g) Adding magnesium stearate,
h) Compressing the mixture into tablets,
i) Coating the tablets.

## Claims

1. A tablet formulation comprises selexipag form III, wherein selexipag form III has a d (0.9) particle size between 1 µm to 150 µm.

2. The tablet formulation according to claim 1, wherein selexipag form III has a d (0.9) particle size between 1 µm to 100 µm.

3. The tablet formulation according to claim 1, wherein the amount of selexipag form III is between 0.05% and 10.0%, preferably between 0.05% and 5.0% by weight of the total tablet.

4. The tablet formulation according to claim 1, wherein further comprises at least one pharmaceutically acceptable excipient selected from the group comprising binders, fillers, disintegrants, lubricants or mixtures thereof.

5. The tablet formulation according to claim 4, wherein binders are selected from the group comprising hydroxypropyl cellulose, polyvinylpyrrolidone, sodium carboxymethyl cellulose, sodium carboxymethyl cellulose, polyethylene glycol, pregelatinized starch, sodium alginate, hydroxypropyl methylcellulose, carboxy methyl cellulose, methyl cellulose, polymethacrylates, methacrylate polymers, polysaccharides, carbomer, poloxamer, polyacrylamide, polyoxyethylene-alkyl ether, polydextrose, polyethylene oxide or mixtures thereof.

6. The tablet formulation according to claim 5, wherein the binder is hydroxypropyl cellulose.

7. The tablet formulation according to claim 6, wherein hydroxypropyl cellulose has a d (0.9) particle size between 15 µm to 250 µm.

8. The tablet formulation according to claim 6, wherein hydroxypropyl cellulose has a d (0.9) particle size between 25 µm to 230 µm, or between 40 µm to 210 µm, or between 50 µm to 190 µm, or between 60 µm to 170 µm, or between 70 µm to 150 µm.

9. The tablet formulation according to claim 5, wherein the amount of binder is between 1.0% and 8.0%, between 2.0% and 6.0% by weight of the total tablet.

10. The tablet formulation according to claim 4, wherein fillers are selected from the group comprising corn starch, microcrystalline cellulose, lactose monohydrate, neutral pellets, calcium sulfate, cellulose, cellulose acetate, compressible sugar, ethylcellulose, fructose, glyceryl palmitostearate, lactitol, lactose, magnesium carbonate, magnesium oxide, maltodextrin, maltose, sodium alginate, sodium chloride, sorbitol, sucrose, sugar spheres, talc, tragacanth, trehalose, xylitol or mixtures thereof.

11. The tablet formulation according to claim 10, wherein fillers are D-mannitol and corn starch.

12. The tablet formulation according to claim 11, wherein D-mannitol having a specific surface area is 1.01 m²/g or more.

13. The tablet formulation according to claim 10, wherein the amount of filler is between 65.0% and 95.0%, between 78.0% and 90.0% by weight of the total tablet.

14. The tablet formulation according to claim 4, wherein disintegrants are selected from the group comprising sodium starch glycolate, low-substituted hydroxypropyl cellulose, croscarmellose sodium, crospovidone, carboxymethyl cellulose, sodium carboxymethyl cellulose, calcium carboxymethyl cellulose, sodium carboxymethyl starch, hydroxymethyl starch or mixtures thereof.

15. The tablet formulation according to claim 4, wherein lubricants are selected from the group comprising magnesium stearate, calcium stearate, sodium stearyl fumarate, potassium stearate, stearic acid, sodium chloride, sodium benzoate, sodium acetate, sodium oleate, polyethylene glycols or mixtures thereof.
